(19) **Europäisches Patentamt**
**European Patent Office**
**Office européen des brevets**

(11) **EP 3 874 277 B1**

(12) **EUROPEAN PATENT SPECIFICATION**

(45) Date of publication and mention
of the grant of the patent:
**02.08.2023 Bulletin 2023/31**

(21) Application number: **19805872.9**

(22) Date of filing: **30.10.2019**

(51) International Patent Classification (IPC):
*G01N 33/68* (2006.01)   *C12Q 1/6869* (2018.01)
*G16B 30/00* (2019.01)   *G16B 40/00* (2019.01)

(52) Cooperative Patent Classification (CPC):
(C-Sets available)
**G01N 33/6818; G16B 30/10; G16B 40/10;**
C12Q 1/6869; C12Q 2537/165      (Cont.)

(86) International application number:
**PCT/EP2019/079736**

(87) International publication number:
**WO 2020/089337 (07.05.2020 Gazette 2020/19)**

(54) **METHOD FOR IDENTIFICATION OF BIOPOLYMERS**

VERFAHREN ZUR IDENTIFIZIERUNG VON BIOPOLYMEREN

PROCÉDÉ POUR L'IDENTIFICATION DE BIOPOLYMÈRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.10.2018 GB 201817786**

(43) Date of publication of application:
**08.09.2021 Bulletin 2021/36**

(73) Proprietor: **Katholieke Universiteit Leuven
3000 Leuven (BE)**

(72) Inventors:
• **HOFKENS, Johan**
**3050 Oud Heverlee (BE)**
• **JANSSEN, Kris**
**9420 Ottergem (BE)**
• **BOUWENS, Arno**
**1050 Brussels (BE)**
• **VITALE, Raffaele**
**1000 Brussels (BE)**
• **DEEN, Jochem**
**1400 Yverdon-les-bains (CH)**

(74) Representative: **Arnold & Siedsma
Bezuidenhoutseweg 57
2594 AC The Hague (NL)**

(56) References cited:
**WO-A1-2011/150475      US-A- 5 701 256**

• **ADAM N. NILSSON ET AL: "Competitive
binding-based optical DNA mapping for fast
identification of bacteria - multi-ligand transfer
matrix theory and experimental applications on
Escherichia coli", NUCLEIC ACIDS RESEARCH,
vol. 42, no. 15, 10 July 2014 (2014-07-10) , pages
e118-e118, XP055557924, ISSN: 0305-1048, DOI:
10.1093/nar/gku556 cited in the application**
• **LENA K. NYBERG ET AL: "Rapid identification of
intact bacterial resistance plasmids via optical
mapping of single DNA molecules", SCIENTIFIC
REPORTS, vol. 6, no. 1, 27 July 2016 (2016-07-27),
XP055557927, DOI: 10.1038/srep30410**

EP 3 874 277 B1

(52) Cooperative Patent Classification (CPC): (Cont.)

C-Sets
C12Q 1/6869, C12Q 2565/631

## Description

## Field of the invention

The invention relates to the field of biopolymer identification, and in particular provides methods for faster identification of highly similar biopolymers with single molecule sensitivity and high specificity.

## Background of the invention

There are many practical applications in medicine, medical diagnostics and biology where there is a need for the identification and quantification of biopolymers such as DNA, RNA and proteins. Biopolymers can be identified by measuring their full monomer sequence. However, for many applications it is not necessary to measure the full monomer sequence in order to identify the biopolymer. Moreover, full monomer sequence determination might not be practically or economically feasible.

Linear analysis of the biopolymer DNA is achieved through fragmentation of the biopolymer to be analyzed. Fragmentation of the source material is followed by various combinations of enzymatic treatments with the aim of duplicating these fragments (i.e. clonal amplification), for example through the use of an enzyme mediated polymerase chain reaction, or adaptation of the fragments for inclusion in monomer readout equipment. The efficiency of enzymatic treatment of DNA fragments in this manner is strongly influenced by the sequence composition of individual fragments, in particular their GC content. Fragment specific efficiency of these treatments will therefore result in over- or under-representation of individual fragments in the monomer level readout data. This bias will negatively impact accurate reconstruction of the original monomer level sequence, ultimately diminishing the ability to correctly identify and quantify biopolymers.

Linear analysis of the biopolymer RNA requires enzyme assisted conversion of the RNA source material to DNA in a 'reverse transcription' reaction, after which methods for linear analysis of DNA can be applied.

Readout of monomer level sequence information follows clonal amplification and adaptation of DNA fragments and can be achieved in various ways. Clonally amplified fragments can be immobilized in flow channels or on the surface of carrier materials facilitating stepwise inclusion of monomers, luminescent monomers, chemically modified monomers or sequence specific tags. Inclusion events are recorded in parallel for large numbers of fragments through the use of optical or electronic instrumentation. Other readout schemes rely on the translocation of suitably adapted sequences through arrays of narrow diameter channels, artificially prepared or protein based narrow diameter holes, i.e. 'nanopores'. Upon translocation of the fragment, optical or electronic signals originating from individual channels or nanopores will be modulated in response to the identity of the monomer at the readout location. Correlation of the signal modulation with respect to fragment translocation allows monomer level reconstruction of the fragment sequence.

All readout schemes share the common characteristic that readout is highly parallelized and that the absolute amount of single monomer readout events is very large and fixed. Modern monomer level sequence determination strategies offer large amounts of information yet at high cost. In addition, the fixed single monomer readout capability requires pooling of large numbers of individual samples to provide sufficient signal for the readout instrumentation. These methods are therefore less applicable for rapid screening or cost effective diagnostic applications.

Linear analysis of protein biopolymers relies on denaturation of the protein to achieve linearization of the peptide sequence. Following denaturation, the full-length peptide sequence is fragmented into smaller poly peptides which are individually purified using e.g. chromatography methods. Short peptide fragments are subsequently degraded in a stepwise manner in such a way as to release individual monomers from the peptide sequence. Released monomers can be analysed using e.g. mass spectrometry methods to allow their identification. To enable single monomer level analysis of the full protein biopolymer sequence, the process of fragmentation and stepwise digestion typically has to be repeated multiple times with differing fragmentation patterns. Alternatively, denatured, linearized and fragmented polypeptides can be translocated through arrays of nanopores. Upon translocation of the fragment, optical or electronic signals originating from individual channels or nanopores will be modulated in response to the identity of the monomer at the readout location. Correlation of the signal modulation with respect to fragment translocation allows monomer level reconstruction of the fragment sequence. Sequence analysis methods relying on stepwise degradation suffer from throughput constraints and require application of highly specialized and sensitive equipment (e.g. mass spectrometry equipment) to identify monomers. Nanopore based methods for protein analysis suffer from the same drawbacks as nanopore based systems for DNA or RNA analysis.

An alternative to sequencing the full monomer sequence is mapping the sequence (see e.g. patent application EP2577275A1). In this method, the physical position of a small monomeric sequence (typically of length 4-8 monomers) are measured over the entire length of the biopolymer. This map is created by attaching labels to the small monomeric sequence (e.g. proteins, fluorescent dyes, charged particles) (Deen et al. (2017) Angew. Chemie - Int. Ed. 56, 5182-5200). The map is read after elongating it on a surface or in solution and measuring the changing signal (fluorescence, charge or topography) with a fluorescence microscope, atomic force microscope (AFM), nanopore or nanocapillary measurements or any other method using electrical or electromagnetic method. Because sequence mapping does not require amplification of the biopolymers to be identified, it has the potential to provide unbiased biopolymer quantification. Moreover, in terms of data quantity, sequence maps are a condensed version of the full monomer sequence. Hence, the reduced data load enables higher read-out and processing speeds.

Existing methods allow to produce and measure sequence maps from biopolymers (EP2577275). However, existing methods for identifying biopolymers from their sequence maps (Valouev et al. (2006) J. Comput. Biol. 13, 442-462; Nilsson et al. (2014) Nucleic Acids Res. 42, e118) lack techniques for improving identification specificity, which is needed for analysing complex mixture samples. Hence, there is a need for methods that are capable of accurately identifying the corresponding biopolymers from the measured sequence maps with both high (ideally single-molecule) sensitivity, and high specificity. Such a method would enable the advantages of sequence mapping over the state of the art for biopolymer identification: no amplification bias, no need to pool samples, reduced data load (no need for sequence analysis at monomer resolution), and higher throughput.

**Summary of the invention**

The invention is defined by the methods according to the appended claims. Systems are disclosed and may be used to practise the invention but are not claimed *per se.*

The invention relates to a biopolymer identification system consisting of a measurement unit capable of reading a serial signal from single biopolymers, and a signal processor identifying the different biopolymers present in a sample. The methods presented in this invention allow unbiased identification of biopolymers with high sensitivity and high specificity, allowing to distinguish between biopolymers with similar monomer sequences. The invention provides methods for, but is not limited to, measuring the abundances of different organisms or viruses in a mixture sample of their nucleic acids. Additionally, the invention provides methods for, but is not limited to, measuring the abundances of different proteins in a mixture sample.

The invention provides methods for rapidly identifying one or more biopolymers in a mixture sample with high (single-molecule) sensitivity, and high specificity. Methods of the invention include producing a serial signal, termed a 'map', from the monomer sequence of the biopolymers, and read-out of the maps by optical, electronic or electromagnetic means.

An objective of the invention is the identification of one or more organisms or viruses from a mixture sample of their nucleic acids. Methods of the invention include the production of a serial signal, termed a map, from the sequence of the nucleic acids, and read-out of the maps by optical, electronic or electromagnetic means.

Another objective of the invention is the identification of proteins from a mixture sample. Methods of the invention include the production of a serial signal, termed a 'map', from the peptide sequence of the proteins, and read-out of the maps by optical, electronic or electromagnetic means.

Methods of the invention include a processor executing software calculating the degree of similarity, termed 'matching score', between measured maps and theoretical maps for each biopolymer in a user-defined set of target biopolymers. This is achieved by the calculation of matching scores as described in the detailed description of the embodiments of the invention.

Another objective of the invention is to perform a nonparametric significance test on the matching scores. Since no statistical model needs to be imposed, this nonparametric method can be generally applied, regardless of the chosen matching score and measurement method. The test determines if a matching score to a target biopolymer is significant. For each measured map, a list of target biopolymers to which it matches significantly is produced.

Another objective of the invention is to improve matching specificity, without sacrificing sensitivity. A shortlist of best matching biopolymers is produced from the list of significant matches. Methods of the invention include a processor executing software performing a second nonparametric significance test on the matching scores. The second test determines if a given matching score is significantly better than a second matching score. The shortlist is thus constructed from the list of significant matches by taking the target biopolymer with the highest matching score and adding only those matches for which the matching score is not significantly lower.

Another objective of the invention is to measure one or more maps in a homogenous sample and identify the biopolymer present in the sample. Another objective of the invention is to measure a number of maps in a heterogeneous mixture sample, determine which biopolymers are present and estimate their abundances.

The above cited objectives are achieved with the present invention which concerns a biopolymer identification system comprising: a sample holder; a measurement unit capable of measuring single molecule biopolymer maps; a processor executing software calculating matching scores for all target biopolymers, a processor executing software performing a nonparametric significance test on the matching scores, a processor executing software creating a shortlist of the best significant matches, and a processor executing software calculating abundances of organisms present in the sample.

The methods of the invention enable various applications. Special embodiments are: compositional analysis of microbial communities for the purpose of monitoring food safety, single or repeated measurement of the composition of microbial communities in or on a patient who might be amenable to, or is undergoing, treatment with antibiotics or probiotics, diagnosis of diseases from known biomarkers based on the composition of microbial communities and detection of a particular strains of microbes.

The invention is further summarized in the following statements (statement 1 corresponding to the claimed invention in its broadest sense):

1. A method for the identification of a biopolymer comprising the steps of:

a. measuring the linear pattern of sequence specific markers on a biopolymer,

b. calculating the similarity score between the measured linear pattern and an expected linear pattern for each individual member of a user-defined set of potential target biopolymers,

c. calculating the significance for each of the obtained similarity scores, thereby obtaining a set of target biopolymers with significant similarity of the expected linear pattern to the measured linear pattern,

d. modifying the expected linear pattern of the set of target biopolymers of step c) by reshuffling marker positions from the expected pattern of said target biopolymers,

e. calculating the similarity score between the measured linear pattern and the modified expected linear pattern of step d) for each individual member of the significant set of potential target biopolymers of step b),

f. calculating the significance for each of the obtained similarity scores, thereby obtaining, compared to the set of target biopolymers of step c, a reduced set of target biopolymers with significant similarity, resulting in improved specificity without loss of sensitivity for the identification of the measured sequence.

2. The method according to statement 1, wherein the reshuffling is performed over the full length of the biopolymer or within windows of the full length of the biopolymer.

3. The method according to statement 1 or 2, where the biopolymer is a nucleic acid.

4. The method according to statement 1 or 2, wherein the biopolymer is a polypeptide.

5. The method according to any one of statements 1 to 4, wherein sequence specific markers are attached to the biopolymer.

6. The method according to statement 5, wherein the sequence specific attachment of markers to the biopolymer is performed by an enzyme or by a chemical modification.

7. The method according to any one of statements 1 to 6, wherein the sequence specific markers are fluorescent molecules.

8. The method according to any one of statements 1 to 7, wherein the sequence specific markers modulate the electrical current across a nanopore upon passage through the nanopore.

9. The method according to any one of statements 1 to 8, wherein the biopolymers are labelled with multiple types of distinguishable markers

10. The method according to any one of statements 1 to 9, wherein multiple recognition sequences are labelled with a marker.

11. The method according to any one of statements 1 to 10, where the sequence specific marker corresponds to a single monomer.

12. The method according to any one of statements 1 to 10, where the sequence specific marker corresponds to multiple monomers.

13. The method according to statement 12, where the sequence specific marker corresponds to 2 to 16 monomers.

14. The method according to any one of statements 1 to 13, wherein the biopolymers are linearized on a surface.

15. The method according to any one of statements 1 to 14, wherein the biopolymers are linearized in nanofluidic or microfluidic channels.

16. The method according to any one of statements 1 to 15, wherein biopolymers are being translocated through a nanopore channel.

17. The method according to any one of statements 1 to 16, wherein the measured linear pattern is the fluorescence intensity along the biopolymer's length measured by optical microscopy.

18. The method according to any one of statements 1 to 17, wherein the measured linear pattern is the fluorescence intensity along the biopolymer's length measured by super-resolution optical microscopy.

19. The method according to statement 1, wherein the measured linear pattern is a list of marker locations along the biopolymer's length measured by localization microscopy.

20. The method according to statement 1, wherein measured linear pattern is the electrical current modulated by sequence specific markers on the biopolymer's monomer sequence measured across a nanopore during the translocation of a biopolymer.

21. The method according to any one of statements 1 to 4 or 8 to 20, wherein no sequence specific markers are attached to the biopolymer and the linear pattern is produced by measuring the electrical current modulation as the biopolymer's monomer sequence is translocated across a nanopore.

22. The method according to any one of statements 1 to 21, wherein multiple linear patterns are measured per biopolymer.

23. The method according to any one of statements 1 to 22, wherein a processor executes software calculating the matching score from the cross-correlation between the measured signal and the theoretical signal for a target biopolymer.

24. The method according to any one of statements 1 to 22, wherein a processor executes software calculating the matching score using a dynamic programming alignment of the measured signal and the theoretical signal for a target biopolymer.

25. The method according to any one of statements 1 to 24 for estimating fractions of the identified biopolymers in a mixture sample.

26. The method according to any one of statements 1 to 24 for compositional analysis of biopolymers of microbial or viral communities for the purpose of monitoring food safety.

27. The method according to any one of statements 1 to 24 for single or repeated measurement of biopolymers of microbial communities in or on a patient who might be amenable to, or is undergoing, treatment with antibiotics or probiotics.

28. The method according to any one of statement 1 to 24 for single or repeated measurement of biopolymers of viral communities in or on a patient.

29. The method according to any one of statements 1 to 24 for diagnosis of diseases from known biomarkers based on the composition of microbial communities and detection of a particular strains of microbes or viruses.

30. The method according to any one of statements 1 to 24 for identification of nucleic acid biopolymers of tumour cells.

## DETAILED DESCRIPTION

Brief description of figures

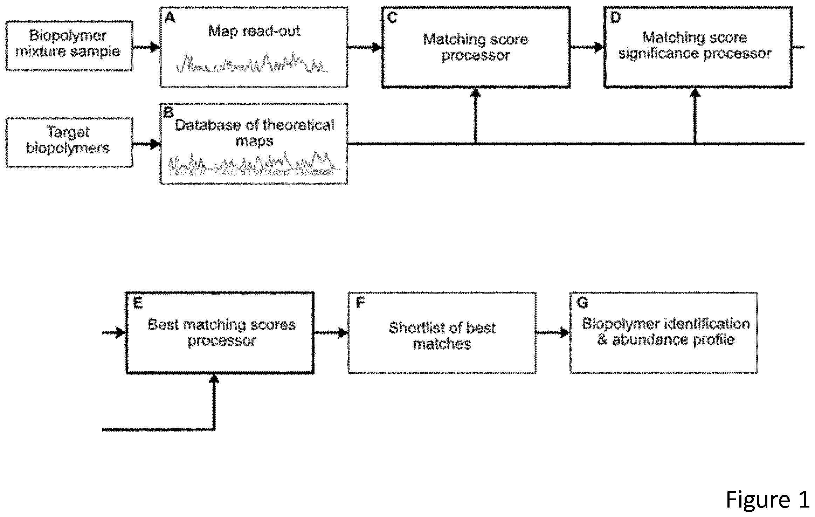

**Figure 1:** Overview of the biopolymer map reader. (A) A biopolymer mixture sample is measured by the map read-out system. (B) Software executed on a processor in the biopolymer map reader system creates a database of theoretical maps for each target biopolymer. (C) Software executed on the matching score processor calculates the matching scores between the measured map and the theoretical maps. (D) The matching score significance processor performs a significance test on the maximum matching scores for all the target species. Only matches passing the significance test are passed on to the best matching scores processor. (E) The best matching scores processor estimates the highest matching score distribution and performs a second significance test to determine which matches are best. (F) In this way, the best matching score processor constructs a shortlist of best matching target species. (G) Finally, after analysing enough biopolymer fragments in the mixture sample, the biopolymer map reader yields the sample's estimated composition and the abundances of the biopolymers in the sample.

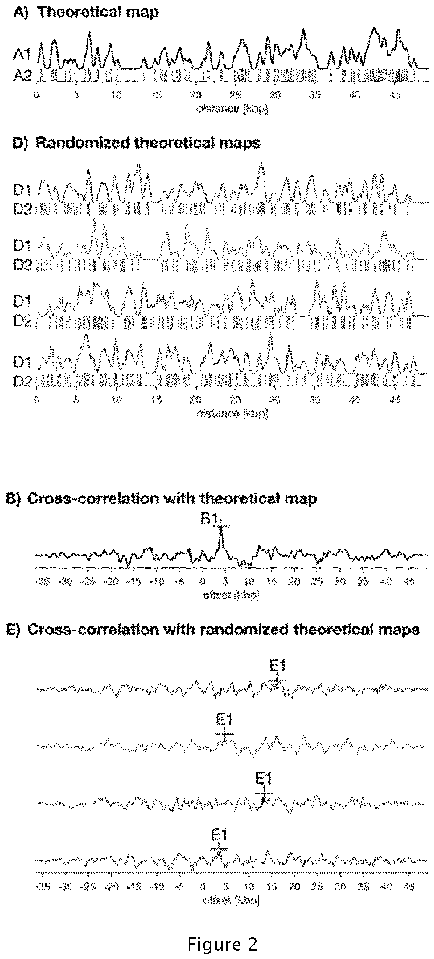

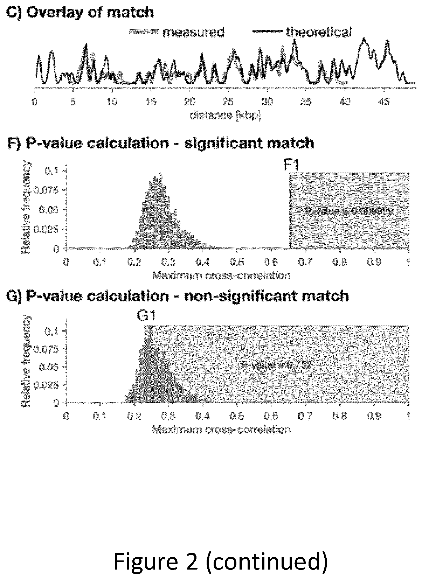

**Figure 2:** In one implementation, the biopolymer maps are measured as signal intensity profiles, which is used in the figure to illustrate the workings of the matching score processor and the matching score significance processor. (A) For each target biopolymer, the matching score processor executes software calculating a theoretical biopolymer map A1, from the expected locations of the labels A2 along the biopolymer. (B) The software executed on the matching score processor calculates the normalized cross-correlation between the measured biopolymer map and the theoretical map for a target biopolymer. (C) By taking the maximum of the cross-correlation to be the matching score, the measured map is aligned to the theoretical map. (D) The matching score significance processor executes software creating reshuffled lists of label locations D2 from the target biopolymer's original list of locations. Next, the matching score significance processor executes software calculating randomized theoretical maps D1 from the reshuffled lists. (E) The matching score significance processor executes software calculating the normalized cross-correlations between the measured biopolymer map and the randomized theoretical maps, from which it takes the maxima E1. These maxima are the randomized matching scores. (F,G) Finally, the matching score significance processor executes software calculating a P-value for the match between the measured map and the theoretical map. (F) If the score for the match between the measured map and the theoretical map (vertical line F1) is higher than most of randomized scores, the match is significant. A significance threshold determines the maximum amount randomized scores that can be higher than the measured score. (G) If the score for the match between the measured map and the theoretical map (vertical line G1) is not higher than most of randomized scores, the match is not significant and is rejected.

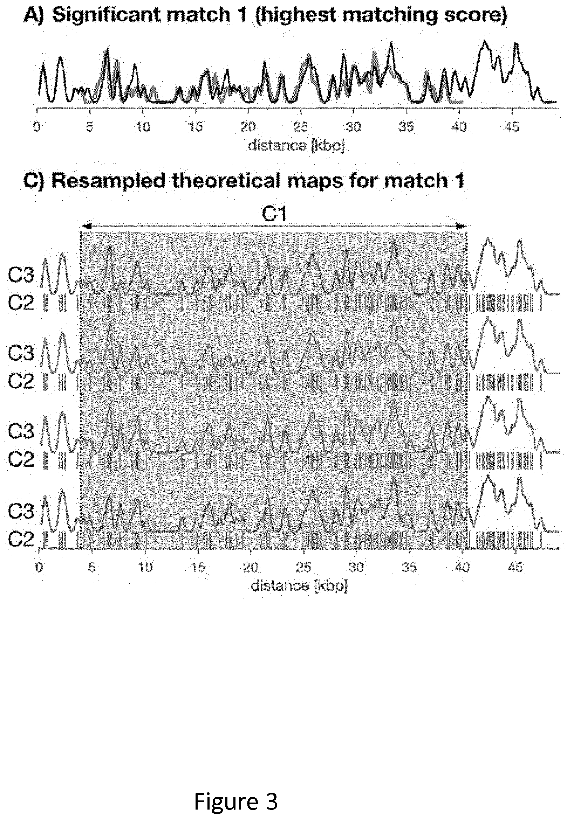

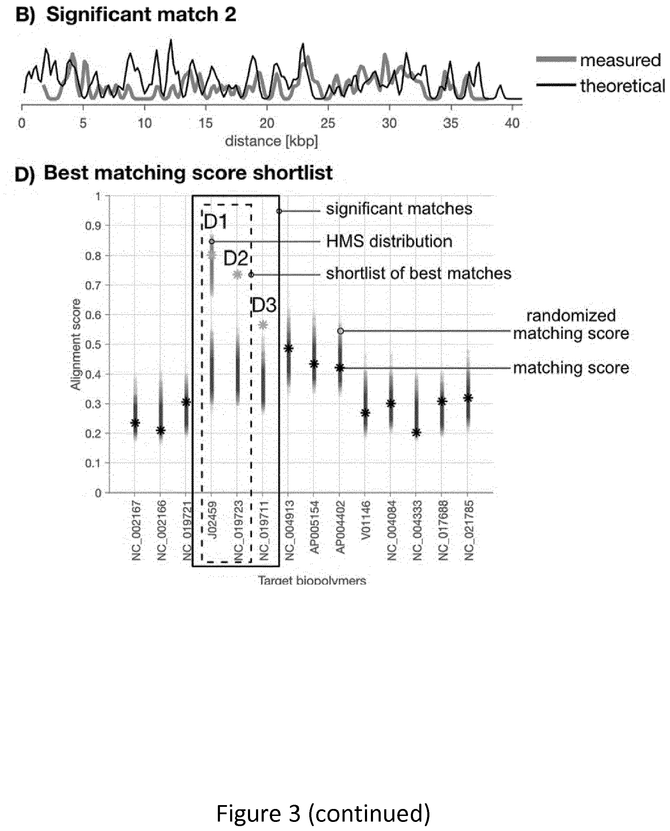

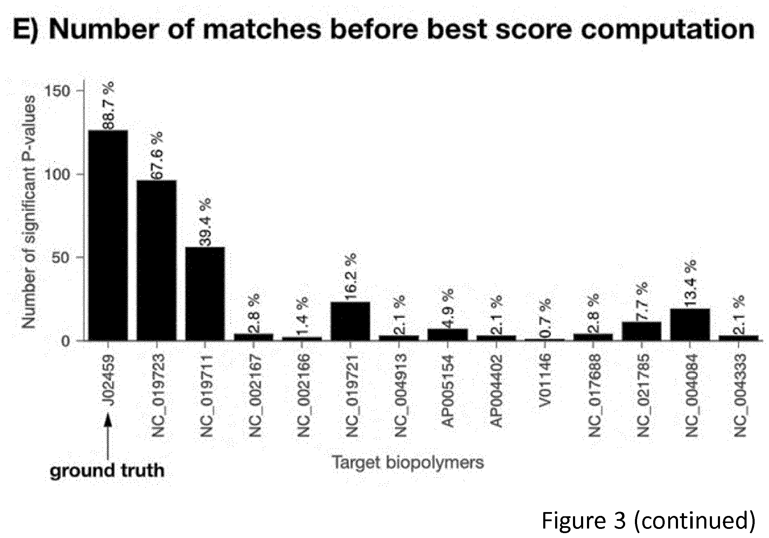

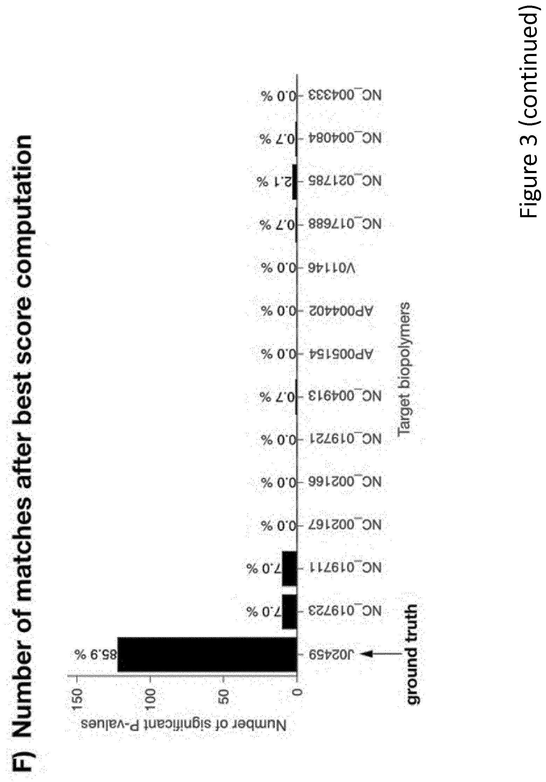

**Figure 3:** The best matching scores processor executes software which creates a shortlist of best matches from the list of significant matches found by the matching score processor. (A,B) Two examples of matches are shown between a measured biopolymer map and two theoretical biopolymer maps. The best matching scores processor executes software finding the match with the highest score (A in the example of this figure) it is added as the first element in the shortlist of best matches. (C) The location of this match within the theoretical map defines a window indicated by the grey shaded area C1. The best matching scores processor executes software estimating the highest matching score (HMS) distribution by randomly removing R labels (for example, R = 2), and/or adding A labels (for example, A = 2) in the window C1, thus creating resampled lists of label locations C2. Next, the best matching scores processor executes software calculating the resampled theoretical maps C3 corresponding to the resampled label location lists C2. For each of these new maps, the matching score is calculated at the same location of the highest matching score. (D) Together, these matching scores make up the HMS distribution. The scores of all other matches, such as matches to other target biopolymers, can now be compared to the HMS distribution. This is done by calculating a second P-value. When a matching score is significantly lower than the highest matching score, it is discarded. In the example depicted in the figure, target biopolymer D3 is significantly lower than D1, as it is lower than the most of the samples of the HMS distribution. On the other hand, when a matching score is not significantly lower than the highest matching score, it is added to the shortlist of best matches. In the example depicted in the figure, D2 is not significantly lower than D1. In the example of the figure, D1 and D2 constitute the best matches shortlist.

**Definitions, terms and elements**

Recognition sequence: A short specific sequence of monomers, usually of 2 to 8 monomers in length, to which a marker is attached.

Markers: The labels attached to the recognition sequence which are detectable by the single molecule reader. For example, fluorescent molecules attached by a methyl transferase enzyme to DNA at the recognition sequence positions. Markers can either be naturally present on the biopolymer or added synthetically.

Map: The serial signal from a single linearized biopolymer due to the markers attached at the recognition sequence positions.

DNA map: A map where the biopolymer is a DNA molecule. The recognition sequence is formed by a short specific sequence of bases.

Theoretical map: The expected map for a target biopolymer and map measurement method. A theoretical map can be obtained from the known monomer sequence of the biopolymer, where all positions of the recognition sequence are marked and the measurement method is simulated. Alternatively, a theoretical map can be synthesized from measurements of the measured maps from pure samples of the biopolymer's maps.

Theoretical DNA map: A theoretical map where the biopolymer is a DNA molecule from an organism. The monomer sequence needed to construct the theoretical DNA map is the organism's DNA sequence. Alternatively, a theoretical DNA map can be synthesized from measurements from pure samples of the organism's DNA maps.

Measured map: The serial signal obtained from a map by the measurement method. Examples include the fluorescence intensity signal along the length of a linearized DNA molecule measured by an optical microscope, a list of the locations of fluorescent molecules along a linearized DNA molecule determined by a localization microscope, and the modulated electronic current across a nanopore when a DNA molecule passes through it.

Processor: Any hardware capable of executing the software described herein. Examples include application-specific integrated circuits (ASICs), field programmable gate arrays (FPGAs), central processing units (CPUs), and graphical processing units (GPUs).

PSF: Point spread function, i.e. the image of a point object.

The invention provides methods for fast identification of biopolymers in mixture samples, including but not limited to genomic nucleic acid samples. The methods provided herein allow the measurement of the abundance of organisms in mixture samples by identifying their genomic DNA.

One class of mixture samples for which the invention can be used are the human microbiomes. Human microbiota are the billions of microorganisms that live inside and on the human body. The various roles of these microorganisms form an important part of normal human physiology. For example, the gut microbiota aid in digestion or protect against disease by competing with and eradicating pathogens.

The study of the human microbiota is a very active area of research, with an increasing number of studies indicating that shifts in the composition of microbiota can predict and cause disease. Indeed, a strong involvement of the microbiome has been found in different diseases such as inflammatory bowel disease, obesity, psoriasis and colon cancer. The clinical potential of a technique allowing the monitoring of microbiota in a cost and time-efficient manner combined with the current inability to correctly measure the microbiome content generates a unique opportunity for technology development.

In principle, microbiota can be uniquely identified by their collective genomes, termed the metagenome. However, analysing the microbiome with current techniques is a very time-consuming, complex, and error-prone task. Indeed, while a screening for the presence of a single bacterium could be done with a simple PCR experiment, this becomes exceedingly complex for simultaneous measurements of mixtures of different species. This diagnostic setup is further complicated when a full overview of the microbiome is required, for example in the case of modulation of the gut microbiota by a probiotic, in biomarker screening or when the clinical efficacy of a treatment needs to be validated in a companion diagnostic.

The current state of the art in microbiome sequencing provides single base resolution of the DNA content yet suffers from a number of inherent limitations. Firstly, the time required to obtain a measured microbiome from a DNA sample ranges from about a week for amplicon sequencing to months for shotgun sequencing. Such long waiting times render applications requiring repeated microbiome sequencing or companion diagnostics impossible or at least impractical. Moreover, sequence alignment of the huge number of small DNA fragments produced by shotgun sequencing is a computationally intensive task and requires access to computer clusters.

Secondly, the faster amplicon sequencing (e.g. 16s rRNA) suffers from a large bias in the measured microbiome content, because the lack of a universal primer introduces an imbalanced PCR amplification. Although standardization of sample treatment protocols may improve cross-reproducibility, this inherent error will never disappear completely.

Additionally, due to the single base resolution of sequencing techniques, the amount of data quickly becomes unmanageable, or at least requires expensive computational resources.

An alternative to base-by-base sequencing of the microbiome is genomic mapping. Genomic mapping does not provide single base resolution, but rather provides long-range information on the DNA sequence by recording distances between specific DNA sequences. It is thus able to surpass repeats, rearrangements and high GC-content (guanine-cytosine content) regions, which are challenging for DNA sequencing, while at the same time greatly reducing the data load, and the computational and sample preparation time.

In genomic mapping, labels are attached to specific short sequence reads on large genomic fragments. The most common way to accomplish this uses DNA Methyltransferases (MTases) [EP2577275A1, (2017); Deen et al. (2017) Angew. Chem. 56, 5182-5200]. Depending on the specific MTases used, labels are attached to a specific base in the recognition sequence (of 2-8 bases) of the enzyme. When fragments are subsequently linearized and measured, they reveal a pattern of labels along the length of the fragment, i.e. a 'DNA map'. For example, when the labels attached by the MTases are fluorescent probes, the DNA map is a pattern of fluorescence intensity.

Besides MTases, other labelling methods can be used to create DNA maps, for example, nicking enzymes, restriction enzymes, binding agents such as netropsin, and Cas9.

In order to match a DNA map to a given organism, it needs to be compared to the theoretical map for that organism, calculated from its known genomic sequence (available from e.g. NCBI's GenBank). Quantitative assessment of the degree of similarity, or 'matching score', between measured and theoretical maps can be achieved in various ways, depending on the DNA map read-out that was used. For example, when using optical microscopy to image the DNA map, the maximum cross-correlation of the intensity profiles of the theoretical convoluted and measured maps can be used as a scoring method (see EP2577275). When using localization microscopy, the matching score can be calculated using the well-known Smith-Waterman alignment algorithm, as implemented for example by Valouev et al. (2006) J. Comput. Biol. 13, 442-462.

While these scoring methods give an indication of the degree of similarity between measured and theoretical maps, there is no obvious way to assess whether a given score is high enough to be judged reliable. This judgment is necessary in order to decide whether a measured DNA map belongs to a certain organism, or whether the high score is due to chance.

One obvious solution could be to assume that the organism with the highest matching score is the correct one. However, there are a number of important problems with this solution. Firstly, when the measured DNA map comes from an organism that is not in the list of organisms to which the matching is performed, one of the organisms in the list would still have the highest matching score. The measured DNA map would therefore wrongly be attributed to this organism. Secondly, when parts of the genome of two organisms are very similar, and the measured DNA map contains such a part of the genome, then this solution would wrongly attribute the DNA map to only one organism. The correct result would have been to assign the DNA map to both organisms. Therefore, this solution would not be adequate for complex heterogeneous samples such as the human microbiota, where the above two cases often occur.

Additionally, the user has no control over the sensitivity versus specificity of this method.

Nilsson et al. presented a different solution to judge the significance of a matching score by calculating a P-value for the scoring values [Nilsson et al. (2014) Nucleic Acids Res. 42, e118]. The P-value is the probability that the score for the match is due to chance. If this probability is low (e.g. below a threshold of 5%), the matching score is said to be statistically significant and therefore reliable. Hence, the P-value can be used as a way to judge the significance of a matching score. In the method of Nilsson et al., the P-value was calculated by imposing a parametric model for the statistical distribution of the matching score, which limits the applicability of their method, and makes it uninterpretable if the model's conditions are not satisfied. One case where the model fails is when the organism's genome is of the same length as a typical DNA map. Another case where the model fails is when an organism's genome contains long repeats.

In the method of Valouev et al., P-values are calculated by random resampling of the theoretical DNA maps. Hence the method of Valouev et al. provides a model-free matching significance test.

P-values calculated following Nilsson et al. or Valouev et al. give an indication about the significance of a match to a single organism by comparing it with matches to randomly resampled versions of that same organism's map. Therefore, in principle, P-values for matches to multiple organisms cannot be compared. Simply taking the match with the lowest P-value as the most reliable match is therefore not an option. One could assign a measured DNA map to all organisms yielding a significant P-value, but this method may have low specificity (many false positive identifications as described in the embodiments). Nilsson et al. proposed an information score (IS) to improve the specificity of their P-value method. In their method, only DNA maps are selected having an IS exceeding an arbitrary threshold. This method rejects measured DNA fragments before calculating their P-values, thereby reducing sensitivity (more false negatives). More importantly, the IS method has an intrinsic bias towards organisms having theoretical DNA maps with higher IS.

It is therefore currently not feasible to reliably (i.e. with high single molecule sensitivity and specificity) estimate abundances of genomic DNA in a heterogeneous mixture sample which is required to identify and quantify organisms in a complex sample such as the human microbiota. The same drawbacks currently apply to the identification and quantification of biopolymers such as RNA and protein in complex mixture samples. This invention provides methods to solve these issues. Below is an overview of the sample processing pipeline for the biopolymer map reader of this invention.

Overview of sample processing pipeline

Briefly, the biopolymer is labelled using the appropriate labelling scheme (e.g. DNA Methyltransferase (MTase) for the biopolymer DNA), followed by sample purification when needed and subjected to the appropriate solution for linear processing (e.g. DNA combing on a surface).

1. Sample preparation

A typical labelling scheme of DNA using DNA MTases starts with extraction of DNA using any desired lysis method. After extraction, a DNA MTase with the desired recognition sequence is added to the DNA mixture with the desired synthetic cofactor in the appropriate conditions for the MTase, as described in Deen et al. (2017) Angew. Chem. Int. Ed. 56, 5182-5200. Following incubation, the MTase is degraded by incubation with a proteinase and the sample is purified using typical DNA purification methods.

2. Measurement of biopolymer maps

Following labelling and purifications, the DNA molecules is then linearized using any conventional elongation methods, for example by DNA combing as described by Deen et al. (2015) ACS Nano 9, 809-816.

Figure 1 depicts an overview of the biopolymer map reader. A biopolymer mixture sample is measured by the map read-out system, as depicted schematically in Figure 1, A. See the examples below for some of the possible embodiments for the map read-out system.

3. Theoretical biopolymer maps

Software executed on a processor in the biopolymer map reader system creates the theoretical maps for each target biopolymer to which the user wishes to match the measured maps, as depicted schematically in Figure 1, B.

The theoretical biopolymer maps are constructed by software executed on a processor in the biopolymer map reader system by first listing the expected label positions along the biopolymer's length, as depicted in Figure 2 A2, and then applying the map reader's transfer function, yielding the theoretical biopolymer map depicted in Figure 2 A1.

In one embodiment where the biopolymers are DNA molecules from organisms, the processor calculates the theoretical maps by first retrieving the known genomic sequence of the organisms (for example from NCBI's GenBank database). For each organism, a list is constructed of the locations of the labelled recognition sequence positions along the length of the genome, as depicted in Figure 2, A2. This list is converted into the theoretical DNA map by applying the DNA map reader's transfer function, as depicted in Figure 2 A1. For each organism, the list of recognition sequence positions and the corresponding theoretical DNA map are stored in the DNA map reader's database, depicted in Figure 1, E.

In one embodiment where the DNA maps are imaged optically by a fluorescence microscope, the transfer function of the DNA map reader is given by the optical transfer function (OTF). The OTF can either be measured experimentally or calculated from the specifications of the imaging system.

Alternatively, the theoretical map is synthesized from previously stored measurements of biopolymer maps from pure samples (i.e. samples containing only one type of biopolymer).

4. Matching score

Software executed on the matching score processor, Figure 1, C, calculates the matching scores (i.e. a metric of signal similarity) between the measured map (Figure 1, A) and the theoretical maps (Figure 1, B).

In one embodiment, the biopolymer maps are measured as signal intensity profiles. This embodiment applies to the case where, for example, the biopolymer is DNA, the markers are fluorescent, and optical microscopy is used to image them. In this case, the software executed on the matching score processor calculates the normalized cross-correlation between the measured DNA map's intensity profile and the theoretical intensity profile (i.e. the theoretical DNA map retrieved from the DNA map reader's database), as depicted in Figure 2 B. The maximum value of the normalized cross-correlation (Figure 2, B1) and its location are transferred to the matching score significance processor, depicted in Figure 1, D. The maximum value is the matching score, termed 'measured matching score'. The location of the maximum yields the estimated position of the measured DNA map within the organism's genome.

In one embodiment, the theoretical maps are created with different stretching factors, as described in EP2577275. This step is required when linearizing of the biopolymers causes the molecule to stretch by an unknown (or imprecisely known) factor. The maximum of the normalized cross-correlation is taken across the cross-correlations for all the stretching factors. In this way, both the estimated position of the measured map within the theoretical map, as well as its estimated stretching factor are found. Other experimental parameters (such as optical resolution, i.e. PSF width) can be optimized and found in the same way.

In another embodiment, the precise locations of labels are determined by single molecule localization microscopy. The single molecule map can be aligned to the theoretical map by a dynamic programming algorithm such as implemented, for example, by Valouev et al. (2006) J. Comput. Biol. 13, 442-462, where the matching score is given by the maximal value of the last label.

5. Matching score significance test

The matching score significance processor, Figure 1, D, executes software implementing the following procedure, illustrated in Figure 2 to test which matching scores are statistically significant.

The list of recognition sequence locations along the biopolymer is constructed from the known monomeric sequence of the biopolymer A2, and each location in the list is replaced by a randomly picked new location within the length of the biopolymer. From the resulting list D2, a new theoretical map is calculated D1. The matching score for this new randomized map is calculated and stored in memory. This matching score, Figure 2, E1, will be called 'randomized matching score'. This procedure is repeated a finite number of times $N_p$, e.g. $N_p$ = 1000. Now, the P-value $p$ is calculated from the equation

$$p = \frac{N+1}{N_p+1},$$

where N is the number of times the randomized matching score (Figure 2 E1) was higher than the measured matching score (Figure 2 F1 & G1). $p = \frac{N+1}{N_p+1}$ is an estimation of the P-value, being the probability that matching to a randomized theoretical map gives a higher score than the measured score, see [Davison & Hinkley, Bootstrap Methods and Their Application. Cambridge University Press, 1997]. If $p$ is lower than a user-defined threshold (e.g. 0.05), the match is called significant (Figure 2 F1). On the other hand, if $p$ is higher than the threshold, the match is called non-significant (Figure 2, G1), and is discarded. Significant matches are transferred to the best matching score processor.

This method was presented by Valouev et al. for the alignment of optical maps using a dynamic programming algorithm based on the Smith Waterman algorithm [Valouev et al. (2006) J. Comput. Biol. 13, 442-462. However, this approach is also applicable to other biopolymer map read-out methods, such as for example wide-field, confocal, and super-resolution microscopy, as well as other electronic or electromagnetic read-out methods.

In one embodiment, all local maxima of the calculated cross-correlation function are analysed individually by the matching score significance processor. All local maxima with a P-value below the threshold are taken into account to determine the best matches by the best match processor.

In one embodiment, the re-shuffling of recognition sequences is done within a fixed-size window, for example of $10^4$ base pairs wide. This allows preserving the local density of recognition sequences in the randomized maps in order to improve specificity when identifying biopolymers.

In one embodiment, where biopolymer maps are measured as intensity traces, a local normalization of the theoretical and/or measured maps is performed by the matching score processor. Local normalization can for example be performed by subtracting the local mean of the maps with a sliding window, and/or dividing by the local standard deviation. Local normalization could be required when the matching score is biased towards regions of high local mean or high local standard deviation.

Best matching scores shortlist

The best matching scores processor Figure 1, E, executes software implementing the following procedure, depicted in Figure 3 F . The matching score significance processor Figure 1, D, transfers only significant matches to the best matching score processor (Figure 3 D). From the list of significant matches, a shortlist of best matches, Figure 1, I, is now constructed to increase the specificity, while having minimal influence on the sensitivity.

First, the match with the highest score (Figure 3, A, and Figure 3, D1) is identified; it is added as the first element in the shortlist of best matches. The location of this match within the theoretical map defines a window, as indicated by the grey shaded area in Figure 3C 1.

The next step is to determine which of the other significant matching scores are significantly lower than the highest score. These matches will then be discarded. The other matches, which are not significantly lower than the highest score, will be added to the shortlist of best matches. This situation occurs due to the stochasticity of the labelling process, which causes excess labels (i.e. false positive labels), and missing labels (i.e. false negative labels). Hence, in order to determine which scores are significantly lower than the highest score, a distribution of the Highest Matching Score (HMS) is needed that approximates the variability due to the labelling process. We will call this distribution the 'HMS distribution'.

The HMS distribution is estimated by randomly removing R labels (for example, $R = 2$), and/or adding A labels (for example, $A = 2$) in the window (Figure 3 C2), thus creating new theoretical maps (Figure 3, C3). We will call the new theoretical maps 'resampled theoretical maps'. A user-defined number $N_b$, for example $N_b = 1000$, of resampled theoretical maps is created in this way. For each of these new maps, the matching score is calculated at the same location of the highest matching score. Together, these matching scores make up the HMS distribution depicted in Figure 3D . The scores of all other matches, such as matches to other target biopolymers, can now be compared to the HMS distribution, as depicted by the stars in Figure 3 D. This is done by calculating a second P-value, termed $b$

$$ b = \frac{N + 1}{N_b + 1}, $$

where N is the number of samples from the HMS distribution that are lower than the tested matching score. $b$ is calculated for all other matches. When $b$ is lower than a user-defined significance level (e.g. 0.05), the corresponding matching score is said to be significantly lower than the highest matching score. When a matching score is significantly lower than the highest matching score, it is discarded (target biopolymer NC_019711 in Figure 3D). On the other hand, when a matching score is not significantly lower than the highest matching score, it is added to the shortlist of best matches (target biopolymer NC_019723 in Figure 3, D2). This situation can occur when the theoretical maps at the matching locations are very similar.

For every measured map in the sample, the shortlist of best matching target biopolymers is constructed by the best matching score processor. The shortlist of best matches is used in the biopolymer map reader's central processor, to assess the content of the sample.

7. Results for homogeneous and heterogeneous mixture samples

Handling mixtures with long biopolymers frequently results in random shearing of the biopolymers. This results in the creation of multiple shorter maps from a long biopolymer. Since different target biopolymers can have different lengths, both the fragment size and the full biopolymer length must be accounted for when calculating the numbers of each type of biopolymer in a sample. Therefore, the equivalent total number of full-length biopolymers is calculated for each target biopolymer, instead of simply counting the total number of matches. Hence, each match contributes $\frac{L_{match}}{L_{genome}}$ genomes, where $L_{match}$ is the length of the matched map, and $L_{genome}$ is the length of the full genome.

If the shortlist of best matches contains more than one biopolymer, the number of biopolymers contributed by those matches can be divided by the number of best matches in the shortlist $N_{best}$. Hence, each match from the shortlist contributes $\frac{L_{match}}{L_{genome}} \frac{1}{N_{best}}$ full-length biopolymers. Alternatively, the matches can be discarded when the shortlist contains more than one biopolymer. This alternative method will result in higher specificity at the cost of some sensitivity.

For every target biopolymer in a heterogeneous mixture sample, the sum total number of full-length biopolymers is found in this way. Finally, relative abundances for each target biopolymer are calculated by dividing by the total number of biopolymers found.

For identification of a homogeneous sample (i.e. the sample is known to contain only one target biopolymer), the same procedure as for a heterogeneous sample can be followed. The target biopolymer for which the most identified full-length biopolymers are found is then taken by the biopolymer map reader as the best estimate of the organism present in the sample.

In one embodiment, the target biopolymers are genomic DNA sequences of a set of target organisms. For example, the target organisms can be a set of bacteria or viruses that are known biomarkers for a certain disease. The mixture sample can be a sample from a microbiome. The methods described herein allow to measure absolute and relative abundances of the target organisms. Hence, the methods of the present invention allow to measure shifts in biomarkers from which medical diagnoses can be made.

**Examples**

**Example 1**

One embodiment of the biopolymer map read-out, Figure 1 A, is based on fluorescence microscopy, and the markers are fluorescent molecules. Fluorescently labelled DNA samples are prepared according to the methods described in EP2577275. The fluorescent DNA maps are imaged by a microscope. Examples of microscopes include a wide-field microscope, a confocal scanning microscope, a super-resolution microscope. The sensitivity and specificity of the method will improve with improved spatial resolution and signal-to-noise ratio of the instrument. Hence, resolution improvement methods can be applied, for example SOFI, structured illumination microscopy (SIM), image scanning microscopy (ISM), or stimulated emission depletion (STED).

In the optical microscope, fluorescence from the labelled DNA strands is excited by the excitation light source, fluorescence emission is collected by the microscope objective, spectrally separated from the excitation light by the dichroic mirror and imaged onto a camera by the tube lens. The image acquired by the camera is transferred to the memory of a processor by the camera interface. Software executed on the processor allows combining multiple fields-of-view of the microscope to cover up to the complete sample area, by moving the sample between image acquisitions. In a preferred embodiment, software on the processor automatically segments the image into the fluorescence intensity patterns (i.e. the DNA maps) of the individual DNA molecules present in the image. The measured DNA maps are transferred to the matching score processor F.

In this example, the DNA map consists of a sampled signal of fluorescence intensity. Matching scores for every DNA map can be calculated by calculating e.g. the normalized cross-correlation, as described above under 'Matching score processor'.

## Example 2

Another embodiment of the biopolymer map read-out, Figure 1 A, is based on fluorescence localization microscopy. In localization microscopy, a movie of the fluorescent biopolymer maps is recorded. The fluorescent markers are made to switch on and off over time, such that they can be localized individually in some of the movie frames, even when they are located closer to each other than the diffraction limit. Localization algorithms executed on the biopolymer reader's processor then construct a list of marker locations for each biopolymer map. Depending on the specific localization algorithm, a single physical fluorescent marker may appear multiple times in the list of marker locations. In this case, a clustering algorithm is executed to ensure every localization corresponds to only one physical fluorescent marker.

In this example, the biopolymer map consists of a list of fluorescent marker positions. Matching scores for every biopolymer map can be calculated either by executing a dynamic programming algorithm e.g. a Smith-Waterman algorithm, or by cross-correlation with the theoretical map, as described above under 'Matching score processor'.

## Example 3

Another embodiment of the biopolymer map read-out, Figure 1 A, is based on measuring electrical current across a nanopore. In this example, biopolymers are labelled at recognition sites with molecules that modulate the electrical current across a nanopore when the biopolymer moves through the nanopore. In this example, the biopolymer map consists of a sampled signal of electrical current. The theoretical biopolymer map is calculated from the known monomeric sequence of the target biopolymer. Matching scores for every biopolymer map are obtained by calculating e.g. the cross-correlation, as described above under 'Matching score processor'.

## Example 4

Another embodiment of the biopolymer map read-out, Figure 1A, is based on measuring electrical current across a nanopore. In this example, the electrical current across the nanopore is modulated by one or more monomers in or near the nanopore while the biopolymer moves through the nanopore. In this example, the biopolymer map consists of a sampled signal of electrical current. The theoretical biopolymer map is calculated from the known monomeric sequence of the target biopolymer. Matching scores for every biopolymer map are obtained by calculating e.g. the cross-correlation, as described above under 'Matching score processor'.

## Example 5

In another embodiment, multiple signals are measured from every DNA map. These multiple signals can be generated, for example, by multi-colour imaging of DNA labelled by complementary methyl transferase enzymes (MTases). In this case, MTases with complementary recognition sequences and spectrally separable fluorescent probes can be used. The DNA map then consists of multiple complementary fluorescence intensity signals, allowing additional information to be extracted from the underlying DNA sequence. The added sequence-specific information improves sensitivity and specificity of our method.

The theoretical and measured DNA maps can be made two-dimensional, where one dimension is the length dimensions of the DNA maps, and the other dimension contains the different signal channels (e.g. the colour channels). When matching the multi-signal DNA map to an organism, a single matching score can be calculated by 2D normalized cross-correlation, while only shifting along the length-dimension. The matching score processor, matching score significance processor and best matching score processor can still be used as described above.

## Example 6

In another embodiment, other methods of attaching markers, for example not based on MTases, can be used. As described by Nilsson et al., competitive binding of probes with different affinities to specific sequences of DNA can be used to make DNA maps [Nilsson et al. (2014) Nucleic Acids Res. 42, e118]. Theoretical maps can be constructed in this case as well, and matching scores can be calculated using the methods described above. Different methods of genomic mapping can be combined to create multi-signal DNA maps. For example, competitive binding and MTase-based genomic mapping can be combined to enhance the performance of identifying organisms.

**Example 7**

To a person skilled in the art, it would obvious that the aforementioned examples can be directly translated to the identification of other biopolymers in complex mixtures. Through a site selective introduction of a label followed by retracing the sequential information from these labels their composition can be retraced and compared to known biopolymers. Examples of such other biopolymers would be proteins and RNA, which can be labelled in both chemical and enzymatic approaches [Anhäuser L, (2017) Curr. Opin. Biotechnol. 48, 69-76; Krall (2018) Nat. Chem. 8, 103-113].

**Claims**

1.  A method for the identification of a biopolymer comprising the steps of:

    a) measuring the linear pattern of sequence specific markers on a biopolymer,
    b) calculating the similarity score between the measured linear pattern and an expected linear pattern for each individual member of a user-defined set of potential target biopolymers,
    c) calculating the significance for each of the obtained similarity scores, thereby obtaining a set of target biopolymers with significant similarity of the expected linear pattern to the measured linear pattern,
    d) modifying the expected linear pattern of the set of target biopolymers of step c) by reshuffling marker positions from the expected pattern of said target biopolymers,
    e) calculating the similarity score between the measured linear pattern and the modified expected linear pattern of step d) for each individual member of the significant set of potential target biopolymers of step b),
    f) calculating the significance for each of the obtained similarity scores, thereby obtaining, compared to the set of target biopolymers of step c, a reduced set of target biopolymers with significant similarity, resulting in improved specificity without loss of sensitivity for the identification of the measured sequence.

2.  The method according to claim 1, wherein the reshuffling is performed over the full length of the biopolymer or within windows of the full length of the biopolymer.

3.  The method according to claim 1 or 2, where the biopolymer is a nucleic acid or a polypeptide.

4.  The method according to any one of claims 1 to 3, wherein sequence specific markers are attached to the biopolymer.

5.  The method according to any one of claims 1 to 4, wherein the sequence specific markers are fluorescent molecules.

6.  The method according to any one of claims 1 to 5, wherein the sequence specific markers modulate the electrical current across a nanopore upon passage through the nanopore.

7.  The method according to any one of claims 1 to 6, wherein the biopolymers are labelled with multiple types of distinguishable markers, or wherein multiple recognition sequences are labelled with a marker.

8.  The method according to any one of claims 1 to 7, wherein the biopolymers are linearized on a surface, or are linearized in nanofluidic or microfluidic channels.

9.  The method according to any one of claims 1 to 8, wherein biopolymers are being translocated through a nanopore channel.

10. The method according to any one of claims 1 to 9, wherein the measured linear pattern is the fluorescence intensity along the biopolymer's length measured by optical microscopy, or wherein the measured linear pattern is the fluorescence intensity along the biopolymer's length measured by super-resolution optical microscopy.

11. The method according to claim 1, wherein the measured linear pattern is a list of marker locations along the biopolymer's length measured by localization microscopy.

12. The method according to claim 1, wherein measured linear pattern is the electrical current modulated by sequence specific markers on the biopolymer's monomer sequence measured across a nanopore during the translocation of a biopolymer.

13. The method according to any one of claims 1 to 3 or 8 to 12, wherein no sequence specific markers are attached to the biopolymer and the linear pattern is produced by measuring the electrical current modulation as the biopolymer's monomer sequence is translocated across a nanopore.

14. The method according to any one of claims 1 to 13, wherein multiple linear patterns are measured per biopolymer.

15. The method according to any one of claims 1 to 14, wherein a processor executes software calculating the matching score from the cross-correlation between the measured signal and the theoretical signal for a target biopolymer or wherein a processor executes software calculating the matching score using a dynamic programming alignment of the measured signal and the theoretical signal for a target biopolymer.

**Patentansprüche**

1.  Ein Verfahren zur Identifizierung eines Biopolymers mit den folgenden Schritten:

    a) Messen des linearen Musters von sequenzspezifischen Markern auf einem Biopolymer,
    b) Berechnen des Ähnlichkeitsergebnisses zwischen dem gemessenen linearen Muster und einem erwarteten linearen Muster für jedes einzelne Element eines benutzerdefinierten Satzes möglicher Ziel-Biopolymere,
    c) Berechnen der Signifikanz für jedes der erhaltenen Ähnlichkeitsergebnisse, wodurch ein Satz von Ziel-Biopolymeren mit signifikanter Ähnlichkeit des erwarteten linearen Musters mit dem gemessenen linearen Muster erhalten wird,
    d) Modifizieren des erwarteten linearen Musters des Satzes von Ziel-Biopolymeren aus Schritt c), indem Markerpositionen aus dem erwarteten Muster der Ziel-Biopolymere umstrukturiert werden,
    e) Berechnen des Ähnlichkeitsergebnisses zwischen dem gemessenen linearen Muster und dem modifizierten erwarteten linearen Muster aus Schritt d) für jedes einzelne Element des signifikanten Satzes möglicher Ziel-Biopolymere aus Schritt b),
    f) Berechnen der Signifikanz für jedes der erhaltenen Ähnlichkeitsergebnisse, wodurch, im Vergleich zu dem Satz von Ziel-Biopolymeren aus Schritt c), ein verringerter Satz von Ziel-Biopolymeren mit signifikanter Ähnlichkeit erhalten wird, was zu einer verbesserten Spezifität ohne Verlust von Sensibilität für die Identifizierung der gemessenen Sequenz führt.

2.  Das Verfahren nach Anspruch 1, wobei das Umstrukturieren über die gesamte Länge des Biopolymers oder innerhalb von Fenstern der gesamten Länge des Biopolymers erfolgt.

3.  Das Verfahren nach Anspruch 1 oder 2, wobei das Biopolymer eine Nukleinsäure oder ein Polypeptid ist.

4.  Das Verfahren nach einem der Ansprüche 1 bis 3, wobei sequenzspezifische Marker an das Biopolymer gebunden sind.

5.  Das Verfahren nach einem der Ansprüche 1 bis 4, wobei die sequenzspezifischen Marker aus fluoreszierenden Molekülen bestehen.

6.  Das Verfahren nach einem der Ansprüche 1 bis 5, wobei die sequenzspezifischen Marker den elektrischen Strom über eine Nanopore bei Durchgang durch die Nanopore modulieren.

7.  Das Verfahren nach einem der Ansprüche 1 bis 6, wobei die Biopolymere mit mehreren Arten unterscheidbarer Marker gekennzeichnet sind, oder wobei mehrere Erkennungssequenzen mit einem Marker gekennzeichnet sind.

8.  Das Verfahren nach einem der Ansprüche 1 bis 7, wobei die Biopolymere auf einer Oberfläche linearisiert sind oder in nanofluidischen oder mikrofluidischen Kanälen linearisiert sind.

9.  Das Verfahren nach einem der Ansprüche 1 bis 8, wobei Biopolymere durch einen Nanoporenkanal hindurch transloziert werden.

10. Das Verfahren nach einem der Ansprüche 1 bis 9, wobei das gemessene lineare Muster die Fluoreszenzstärke entlang der durch optische Mikroskopie gemessene Länge des Biopolymers darstellt, oder wobei das gemessene lineare Muster die Fluoreszenzstärke entlang der durch optische Superauflösungsmikroskopie gemessenen Länge des Biopolymers darstellt.

11. Das Verfahren nach Anspruch 1, wobei das gemessene lineare Muster eine Liste von Markerpositionen entlang der durch Lokalisationsmikroskopie gemessenen Länge des Biopolymers darstellt.

12. Das Verfahren nach Anspruch 1, wobei ein gemessenes lineares Muster den durch sequenzspezifische Marker auf der Monomersequenz des Biopolymers modulierten elektrischen Strom darstellt, der über eine Nanopore während der Verlagerung eines Biopolymers gemessen wird.

13. Das Verfahren nach einem der Ansprüche 1 bis 3 oder 8 bis 12, wobei keine sequenzspezifischen Marker an das Biopolymer gebunden sind und das lineare Muster durch Messen der Modulation des elektrischen Stroms erzeugt wird, wenn die Monomersequenz des Biopolymers über eine Nanopore transloziert wird.

14. Das Verfahren nach einem der Ansprüche 1 bis 13, wobei mehrere lineare Muster pro Biopolymer gemessen werden.

15. Das Verfahren nach einem der Ansprüche 1 bis 14, wobei ein Prozessor eine Software ausführt, die das übereinstimmende Ergebnis aus der Kreuzkorrelation zwischen dem gemessenen Signal und dem theoretischen Signal für ein Ziel-Biopolymer berechnet, oder wobei ein Prozessor eine Software ausführt, die das übereinstimmende Ergebnis mithilfe einer dynamischen Programmierungsangleichung des gemessenen Signals und des theoretischen Signals für ein Ziel-Biopolymer berechnet.

**Revendications**

1. Procédé pour l'identification d'un biopolymère comprenant les étapes consistant à :

   a) mesurer le motif linéaire de marqueurs spécifiques à une séquence sur un biopolymère,
   b) calculer le score de similarité entre le motif linéaire mesuré et un motif linéaire attendu pour chaque élément individuel d'un ensemble défini par utilisateur de biopolymères cibles potentiels,
   c) calculer la pertinence pour chacun des scores de similarité obtenus, en obtenant ainsi un ensemble de biopolymères cibles présentant une similarité significative du motif linéaire attendu avec le motif linéaire mesuré,
   d) modifier le motif linéaire attendu de l'ensemble de biopolymères cibles de l'étape c) en remaniant des positions de marqueurs à partir du motif attendu desdits biopolymères cibles,
   e) calculer le score de similarité entre le motif linéaire mesuré et le motif linéaire attendu modifié de l'étape d) pour chaque élément individuel de l'ensemble significatif de biopolymères cibles potentiels de l'étape b),
   f) calculer la pertinence pour chacun des scores de similarité obtenus, en obtenant ainsi, par comparaison à l'ensemble de biopolymères cibles de l'étape c, un ensemble réduit de biopolymères cibles présentant une similarité significative, résultant en une spécificité améliorée sans perte de sensibilité pour l'identification de la séquence mesurée.

2. Procédé selon la revendication 1, dans lequel le remaniement est effectué sur la longueur entière du biopolymère ou dans des fenêtres sur la longueur entière du biopolymère.

3. Procédé selon la revendication 1 ou 2, où le biopolymère est un acide nucléique ou un polypeptide.

4. Procédé selon l'une quelconque des revendications 1 à 3, dans lequel des marqueurs spécifiques à une séquence sont attachés au biopolymère.

5. Procédé selon l'une quelconque des revendications 1 à 4, dans lequel les marqueurs spécifiques à une séquence sont des molécules fluorescentes.

6. Procédé selon l'une quelconque des revendications 1 à 5, dans lequel les marqueurs spécifiques à une séquence modulent le courant électrique à travers un nanopore lors du passage à travers le nanopore.

7. Procédé selon l'une quelconque des revendications 1 à 6, dans lequel les biopolymères sont marqués avec de multiples types de marqueurs distinguables, ou dans lequel de multiples séquences de reconnaissance sont marquées avec un marqueur.

8. Procédé selon l'une quelconque des revendications 1 à 7, dans lequel les biopolymères sont linéarisés sur une surface, ou sont linéarisés dans des canaux nanofluidiques ou microfluidiques.

9. Procédé selon l'une quelconque des revendications 1 à 8, dans lequel des biopolymères sont transloqués à travers un canal nanoporeux.

10. Procédé selon l'une quelconque des revendications 1 à 9, dans lequel le motif linéaire mesuré est l'intensité de fluorescence le long de la longueur du biopolymère mesurée par microscopie optique, ou dans lequel le motif linéaire mesuré est l'intensité de fluorescence le long de la longueur du biopolymère mesurée par microscopie optique à super résolution.

11. Procédé selon la revendication 1, dans lequel le motif linéaire mesuré est une liste d'emplacements de marqueurs le long de la longueur du biopolymère mesurés par microscopie de localisation.

12. Procédé selon la revendication 1, dans lequel le motif linéaire mesuré est le courant électrique modulé par des marqueurs spécifiques à une séquence sur la séquence de monomères du biopolymère mesuré à travers un nanopore pendant la translocation d'un biopolymère.

13. Procédé selon l'une quelconque des revendications 1 à 3 ou 8 à 12, dans lequel aucun marqueur spécifique à une séquence n'est attaché au biopolymère et le motif linéaire est produit en mesurant la modulation de courant électrique lorsque la séquence de monomères du biopolymère est transloquée à travers un nanopore.

14. Procédé selon l'une quelconque des revendications 1 à 13, dans lequel de multiples motifs linéaires sont mesurés par biopolymère.

15. Procédé selon l'une quelconque des revendications 1 à 14, dans lequel un processeur exécute un logiciel calculant le score de correspondance à partir de la corrélation croisée entre le signal mesuré et le signal théorique pour un biopolymère cible ou dans lequel un processeur exécute un logiciel calculant le score de correspondance à l'aide d'un alignement de programmation dynamique du signal mesuré et du signal théorique pour un biopolymère cible.

Figure 1

Figure 2

## C) Overlay of match

## F) P-value calculation - significant match

## G) P-value calculation - non-significant match

# Figure 2 (continued)

Figure 3

Figure 3 (continued)

E) Number of matches before best score computation

Figure 3 (continued)

EP 3 874 277 B1

## F) Number of matches after best score computation

Figure 3 (continued)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2577275 A1

- EP 2577275 A

**Non-patent literature cited in the description**

- **DEEN et al.** *Angew. Chemie - Int. Ed.,* 2017, vol. 56, 5182-5200
- **VALOUEV et al.** *J. Comput. Biol.,* 2006, vol. 13, 442-462
- **NILSSON et al.** *Nucleic Acids Res.,* 2014, vol. 42, e118
- **DEEN et al.** *Angew. Chem.,* 2017, vol. 56, 5182-5200

- **DEEN et al.** *Angew. Chem. Int. Ed.,* 2017, vol. 56, 5182-5200
- **DEEN et al.** *ACS Nano,* 2015, vol. 9, 809-816
- **DAVISON ; HINKLEY.** Bootstrap Methods and Their Application. Cambridge University Press, 1997
- **ANHÄUSER L.** *Curr. Opin. Biotechnol.,* 2017, vol. 48, 69-76
- **KRALL.** *Nat. Chem.,* 2018, vol. 8, 103-113